(19) Europäisches Patentamt
European Patent Office
Office européen des brevets

(11) EP 2 190 289 B1

(12) **EUROPEAN PATENT SPECIFICATION**

(45) Date of publication and mention of the grant of the patent:
**01.05.2013 Bulletin 2013/18**

(21) Application number: **08803041.6**

(22) Date of filing: **15.08.2008**

(51) Int Cl.:
***A01N 43/80*** *(2006.01)* ***A01N 47/12*** *(2006.01)*
***A01N 47/18*** *(2006.01)* ***A01N 53/12*** *(2006.01)*
***A01N 47/42*** *(2006.01)* ***A01P 7/00*** *(2006.01)*
***A61K 31/42*** *(2006.01)*

(86) International application number:
**PCT/EP2008/060732**

(87) International publication number:
**WO 2009/024541 (26.02.2009 Gazette 2009/09)**

(54) **ISOXAZOLINE COMPOSITIONS AND THEIR USE AS ANTIPARASITICS**
ISOXAZOLINVERBINDUNGEN UND IHRE VERWENDUNG ALS ANTIPARASITIKA
COMPOSTIONS D'ISOXAZOLINE ET LEUR UTILISATION EN TANT QU'ANTIPARASITAIRES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **17.08.2007 US 956448 P**
**17.08.2007 EP 07016152**
**21.12.2007 EP 07150309**
**14.07.2008 US 80444**

(43) Date of publication of application:
**02.06.2010 Bulletin 2010/22**

(60) Divisional application:
**12188400.1 / 2 545 777**

(73) Proprietor: **Intervet International B.V.**
**5831 AN Boxmeer (NL)**

(72) Inventors:
- **HECKEROTH, Anja Regina**
**D-55270 Schwabenheim (DE)**
- **LUTZ, Jürgen**
**D-55270 Schwabenheim (DE)**
- **MERTENS, Christina**
**D-45219 Essen (NL)**
- **WILLIAMS, Heike**
**D-55270 Schwabenheim (DE)**
- **ZOLLER, Hartmut**
**D-55270 Schwabenheim (DE)**
- **MITA, Takeshi**
**Chiba (JP)**

(74) Representative: **Stumm, Karin et al**
**Merck Sharp & Dohme Ltd.**
**Hertford Road**
**Hoddesdon, Hertfordshire**
**EN11 9BU (GB)**

(56) References cited:
**WO-A1-2009/003075 US-A1- 2007 066 617**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 190 289 B1

## Description

FIELD OF THE INVENTION

This invention relates to methods for controlling parasitic infestations of animals and their environments, and, more particularly, to methods using isoxazolines to control parasites in or on animals as well as treat parasitoses of animals. The isoxazolines include 4-(isoxazolinyl)-benzamides (specifically, substituted 4-(5-(halomethyl)-5-phenyl-isoxazolin-3-yl)-benzamides) and 4-(isoxazolinyl)-benzothioamides (specifically, substituted 4-(5-(halomethyl)-5-phenyl-isoxazolin-3-yl)-benzothioamides). This invention also relates to compositions comprising the isoxazolines for use in such methods, the use of the isoxazolines to make medicaments for use in such methods. This invention further relates to the use of the isoxazolines as medicaments, particularly medicaments that can be used in the above-referenced methods.

BACKGROUND OF THE INVENTION

A number of pests and parasites are known to infest warm-blooded animals. These pests and parasites can be great nuisances to both the animals and their owners. For example, virtually all companion and livestock animals can be affected by ectoparasites, such as ticks, mites, lice, and fleas. Ectoparasites tend to irritate the animals, and also can cause clinical disease and adverse sub-clinical conditions, either by themselves or by carrying vector-transmitted pathogens. To date, various treatments have been developed to control ectoparasites on warm-blooded animals. Both WO2009/003075 (prior art under Article 54 (3) EPC) and US 2007/0066617 describe the use of isoxazoline compounds e.g. compound of Formula 11-1 for controlling ecto- or endoparasites on mammals. None of these documents discloses the topical administration of the compound of formula 11-1 at a bi-monthly, quartely, half-yearly, or longer frequency to the animal. Nevertheless, a need continues to exists for compositions (and methods for their use) that are bioavailable, can provide contact or systemic activity, are potently efficacious, have a quick onset of activity, have a long duration of activity, and/or are safe to the animal recipients and/or their human owners. This invention addresses this need.

SUMMARY OF THE INVENTION

Briefly, this invention is generally directed to isoxazoline compositions (particularly 4-(isoxazolinyl)-benzamide compositions (also known as, for example, "4-(4,5-dihydroisoxazole-3-yl)-benzoic acid amide" compositions) and their use to control ectoparasites in or on warm-blooded animals. In accordance with this invention, it has been discovered that these compositions generally show desirable bioavailability, and can provide contact and/or systemic activity. Many of the compositions also provide desirable safety profiles toward the warm-blooded animal recipients and/or their owners. In addition, it has been discovered that a single administration of such compositions generally provides potent activity against one or more ectoparasites, while also tending to provide fast onset of activity, long duration of activity, and/or desirable safety profiles.

This invention, therefore, is directed, in part, to a method for controlling ectoparasitic infestation of an animal. The method comprises administering isoxazoline, a salt of the isoxazoline, or a solvate of the isoxazoline or salt to the animal. The isoxazoline according to the invention corresponds in structure to Formula 11-1.

This invention also is directed, in part, to a method for treating parasitoses of an animal. This method comprises administering an above-described isoxazoline, salt, or solvate to the animal.

This invention also is directed, in part, to a method for controlling a ectoparasitic infestation in an environment that is occupied (periodically or continuously) by an animal (e.g., a companion animal, such as a cat or dog). This method comprises administering an above-described isoxazoline, salt, or solvate to the animal.

This invention also is directed, in part, to a use of an above-described isoxazoline, salt, or solvate as a medicament.

This invention also is directed, in part, to a use of an above-described isoxazoline, salt, or solvate to make a medicament for the treatment of parasitoses of an animal.

This invention also is directed, in part, an above-described isoxazoline, salt, or solvate to treat parasitoses of an animal.

This invention also is directed, in part, to a parasiticidal composition for use with an animal. The composition comprises an above-described isoxazoline, salt, or solvate in an amount that is effective to control an ectoparasitic infestation when the composition is administered to the animal. The composition also comprises an excipient (*i.e.*, it comprises at least one excipient).

Further benefits of Applicants' invention will be apparent to one skilled in the art from reading this specification.

BRIEF DESCRIPTION OF THE DRAWING

Figure **1** shows the mean plasma concentration of Compound 11-1 during the study in **Example 4**, which assesses the efficacy of Compound 11-1 against cat fleas (*Ctenocephalides felis*) and brown dog ticks (*Rhipicephalus sanguineus*) in dogs. In **Figure 1,** the "PO" data refers to **Group A** (1 mg/kg body weight Compound 11-1 in the form of a tablet for oral administration); the "SC" data refers to **Group B** (1 mg/kg body weight Compound 11-1 in the form of an injectable solution for subcutaneous administration); the "TOP W/ ENH" data refers to **Group C** (1 mg/kg body weight Compound 11-1 in the form of a topical spot-on solution containing an absorption enhancer); the "TOP W/ ENH & SPREAD" data refers to **Group D** (1 mg/kg body weight Compound 11-1 in the form of a topical spot-on solution containing an absorption enhancer and spreading agent); and the "TOP W/ ETHYL LACTATE" data refers to **Group E** (1 mg/kg body weight Compound 11-1 in the form of a topical spot-on solution containing ethyl lactate as a solvent).

DETAILED DESCRIPTION OF PREFERRED EMBODIMENTS

This detailed description of preferred embodiments is intended only to acquaint others skilled in the art with Applicants' invention, its principles, and its practical application so that others skilled in the art may adapt and apply the invention in its numerous forms, as they may be best suited to the requirements of a particular use. This detailed description and its specific examples, while indicating preferred embodiments of this invention, are intended for purposes of illustration only. This invention, therefore, is not limited to the preferred embodiments described in this specification, and may be variously modified.

The isoxazoline used in accordance with the current invention is the following:

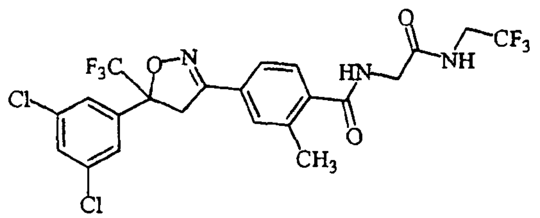

**(11-1).**

The chemical name for this isoxazoline is 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide. It can be found in, for example, CAS RN [864731-61-3]. It has been discovered in accordance with this invention that Compound 11-1 exhibits particularly beneficial *in vivo* results with respect to duration of flea inhibition using one of various routes of administration, including topical, oral, or subcutaneous. *See, e.g.,* Example 3 below.

*I. Isomers*

The isoxazoline used in this invention generally can have two or more conformational structures. At minimum, for example, the isoxazoline comprise a chiral (or asymmetric) carbon at the 5-position of the isoxazoline ring. In some embodiments, for example, the chiral carbon has a left-handed (or "S" or "sinister") configuration. An example of such a compound is:

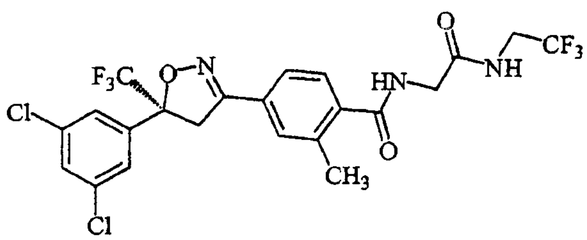

**(17-1).**

In other embodiments, the chiral carbon has a right-handed (or "R" or "rectus") configuration. An example of such a compound is:

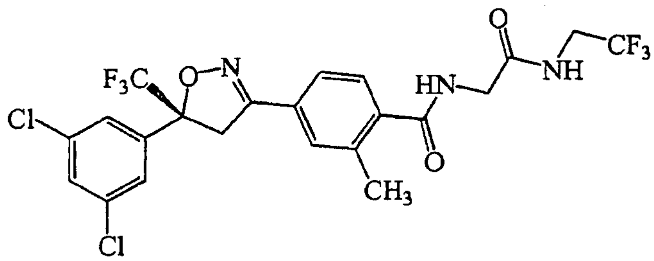

(11-1R).

A specific isomer often can be isolated from the corresponding racemic mixture (or a salt thereof) using, for example, chiral high performance liquid chromatography (HPLC) techniques. Such a technique is illustrated in **Example 5** below for isolating the R and S enantiomers of racemic Compound 11-1. In some instances when an isomer is difficult to separate, a more-easily-isolatable derivative of the isomer is isolated from the corresponding derivative racemic mixture (or a salt thereof), and then converted to the isomer. Alternatively, a specific isomer often can be directly synthesized from, for example, an optically pure starting material.

In some embodiments, the ratio of one enantiomer (e.g., Compound 17-1) to another enantiomer (e.g., Compound 11-1R) in the pharmaceutical composition used with this invention is greater than 1:1. In some instances, for example, the ratio is greater than about 70:30, greater than about 85:15, greater than about 90:10, greater than about 95:5, greater than about 98:2, or greater than about 99:1.

In some embodiments, the concentration of one enantiomer (e.g., Compound 17-1) in the composition (or, more typically, a precursor composition) is greater than about 50% (by weight). In some such embodiments, for example, the concentration is greater than about 70% (by weight), greater than about 85% (by weight), greater than about 90% (by weight), greater than about 95% (by weight), greater than about 98% (by weight), greater than about 99% (by weight), or greater than about 99.5% (by weight).

Unless otherwise stated, a isoxazoline structure that does not indicate a particular conformation is intended to encompass compositions of all the possible conformational isomers of the isoxazoline, as well as compositions comprising fewer than all (e.g., just one of) the possible conformational isomers.

*J. Salts of the isoxazolines*

As noted above, many isoxazolines used with this invention may be in the form of a salt. A salt may be advantageous due to one or more of its physical properties, such as pharmaceutical stability in differing temperatures and humidities; crystalline properties; and/or a desirable solubility in water, oil, or other solvents. Acid and base salts typically can be formed by, for example, mixing a compound with an acid or base, respectively, using various known methods in the art. In general, when the salt is intended to be administered *in vivo* (*i.e.,* to an animal) for a therapeutic benefit, the salt preferably is pharmaceutically acceptable.

In some instances, a base addition salt of a isoxazoline of **Formula 11-1** can be prepared by reacting the isoxazoline with an approximately stoichiometric amount of an inorganic or organic base, typically a strong inorganic or organic base. Examples of base addition salts may include, for example, metallic salts, and organic salts. Metallic salts, in particular, include alkali metal (group Ia, e.g., lithium, sodium, or potassium) salts, alkaline earth metal (group IIa, e.g., barium, calcium, and magnesium) salts, heavy metal (e.g., zinc and iron) salts, and other physiologically acceptable metal salts. Such salts may be made from calcium, lithium, magnesium, potassium, sodium, and zinc. For example, a free acid isoxazoline may be mixed with sodium hydroxide to form such a base addition salt.

In some instances, an acid addition salt of a isoxazoline of **Formula 11-1** can be prepared by reacting the isoxazoline with an approximately stoichiometric amount of an inorganic or organic acid. Examples of contemplated inorganic acids for making pharmaceutically acceptable salts include hydrochloric, hydrobromic, hydroiodic, nitric, carbonic, sulfuric, and phosphoric acid. Examples of often suitable organic acids for making pharmaceutically acceptable salts generally include, for example, aliphatic, cycloaliphatic, aromatic, araliphatic, heterocyclic, carboxylic, and sulfonic classes of organic acids. Specific examples of organic acids include cholic, sorbic, lauric, acetic, trifluoroacetic, formic, propionic, succinic, glycolic, gluconic, digluconic, lactic, malic, tartaric acid, citric, ascorbic, glucuronic, maleic, fumaric, pyruvic, aspartic, glutamic, aryl carboxylic acid (e.g., benzoic), anthranilic acid, mesylic, stearic, salicylic, p-hydroxybenzoic, phenylacetic, mandelic, embonic (pamoic), alkylsulfonic (e.g., ethanesulfonic), arylsulfonic (e.g., benzenesulfonic), pantothenic, 2-hydroxyethanesulfonic, sulfanilic, cyclohexylaminosulfonic, β-hydroxybutyric, galactaric, galacturonic, adipic, alginic, butyric, camphoric, camphorsulfonic, cyclopentanepropionic, dodecylsulfic, glycoheptanoic, glycerophosphic, heptanoic, hexanoic, nicotinic, 2-naphthalesulfonic, oxalic, palmoic, pectinic, 3-phenylpropionic, picric, pivalic, thiocyanic, tosylic, and undecanoic acid.

In some instances, an organic salt of a isoxazoline of **Formula** 11-1 may be made by, for example, quatemizing

a basic nitrogen-containing group on the isoxazoline with an agent such as a $C_1$-$C_6$-alkyl halide (e.g., methyl, ethyl, propyl, and butyl chlorides, bromides, or iodide), dialkyl sulfate (e.g., dimethyl, diethyl, dibuytl, or diamyl sulfate), long chain halide (e.g., decyl, lauryl, myristyl, and stearyl chlorides, bromides, and iodide), arylalkyl halide (*e.g*., benzyl and phenethyl bromide), and the like.

It should be understood that the counterion of an acid or base salt may, in some instances, be optically active (e.g., D-lactate and L-lysine salts) or racemic (e.g., DL-tartrate and DL-arginine salts).

*K. Solvates of the isoxazolines*

In some instances, the isoxazolines of **Formula 11-1** are in the form of stable complexes with solvent molecules that remain intact after the non-complexed solvent molecules are removed from the compounds. These complexes generally are referred to as "solvates." In some instances, the solvate will be capable of isolation, for example when one or more solvent molecules are incorporated into the crystal lattice of the crystalline solid. A "solvate" encompasses both solution-phase and isolatable solvates. Examples of suitable solvates include ethanolates, methanolates, and the like. A "hydrate" is a solvate wherein the solvent molecule is water. A solvate intended to be used *in vivo* preferably is pharmaceutically acceptable.

*L. Prodrugs of the isoxazolines*

It is contemplated that prodrugs of the isoxazoline of **Formula 11-1** may be used with methods of this invention. As used in this patent, the term "prodrug" is a compound that is convertible in use *(e.g., in vivo)* by metabolic means or another processes (e.g., hydrolysis) to a isoxazoline of **Formula 11-1**. In some embodiments, delivery of the isoxazoline in a prodrug form achieves improved delivery of the isoxazoline by improving its physicochemical or pharmacokinetic properties by, for example, enhancing systemic absorption, delaying clearance, or delaying breakdown *in vivo.*

***II. Preparation of isoxazolines of Formula (I)***

Methods for preparing isoxazolines are known in the art. Methods for preparing various isoxazolines of **Formula 11-1** for example, have been discussed in US Patent Publ. No. US2007/0066617 Int'l Patent Publ. No. WO2007/026965 and Japanese Patent Appl. Publ. No. JP2007/308471. For example, US Patent Publ. No. US2007/0066617 discusses the preparation of Compound 11-1 at Example 21 on page 72. addition, methods for preparing various isoxazolines also are discussed in, for example, Int'l Patent Publ. No. WO 2005/085216 Int'l Patent Appl. No. PCT/JP/2008/054096 European Patent Appl. Publ. No. EP1932836 and Japanese Patent Appl. Publ. No. JP2008/133242.

***III. Treatment Methods Using a Composition of this Invention***

The isoxazolines of **Formula 11-1** generally may be used to control ectoparasites on animals, and, in turn, diseases directly caused by such ectoparasites and/or diseases caused by pathogens carried by such ectoparasites. It is contemplated that the composition may be used to treat a range of animals, especially warm-blooded animals. Such warm-blooded animals include, for example, mammals. Mammals include, for example, humans. Other mammals include, for example, farm or livestock mammals *(e.g.,* swine, bovines, sheep, goats, etc.), laboratory mammals *(e.g.,* mice, rats, jirds, etc.), companion mammals *(e.g.,* dogs, cats, equines, etc.), fur-bearing animals *(e.g.,* minks, foxes, chinchillas, rabbits, etc.), and wild and zoo mammals *(e.g.,* buffalo, deer, etc.). In some embodiments, the compositions are used to treat canines *(e.g.,* dogs, such as, for example, pure-bred and/or mongrel companion dogs, show dogs, working dogs, herding dogs, hunting dogs, guard dogs, police dogs, racing dogs, and/or laboratory dogs). In other embodiments, the compositions are used to treat felines *(e.g.,* domestic cats). It is contemplated that the compositions also are suitable to treat non-mammals, such as birds *(e.g.,* turkeys, chickens, geese, ducks, parrots, etc.). It is also contemplated that such compositions may be useful to treat cold-blooded animals as well, such as, for example, fish (e.g., salmon, trout, koi, etc.).

It has been discovered in accordance with this invention that the isoxazolines **Formula 11-1** are generally of particular value for controlling ectoparasites, i.e., arthropods that are injurious to, or spread or act as vectors of diseases in, warm-blooded animals. The isoxazolines are generally beneficial for controlling various lifecycle stages of parasites, including egg, nymph, larvae, juvenile, and adult stages. Ectoparasites (generally insect and acarid pests) include the following.

A. **Biting insects.** These include, for example, migrating diperous larvae, such as, for example, *Hypoderma* sp. in cattle, *Gastrophilus* in horses, and *Cuterebra* sp. in rodents; biting flies, such as, for example, bloodsucking adult flies (*e.g.,* the horn fly *(Haematobia irritans),* horse flies *(Tabanus* spp.), stable flies *(Stomoxys calcitrans*), black

flies (*Simulium* spp.), deer flies (*Chrysops* spp.), louse flies *(Melophagus ovinus),* tsetse flies *(Glossina* spp.)); parasitic fly maggots, such as, for example, bot flies (*Oestrus ovis* and *Cuterebra* spp.), the blow flies *(Phaenicia* spp.), screwworms *(Cochliomyia hominivorax),* cattle grubs (*Hypoderma* spp.), and fleeceworms; and mosquitoes, such as, for example, *Culex* spp., *Anopheles* spp., and *Aedes* spp.

B. **Mites**. These include:

i. *Mesostigmata* spp., such as mesostigmatids, which include chicken mites (*Dermanyssus gallinae*).
ii. *Astigmata* spp., such as itch or scab mites, which include *Sarcoptidae* spp. *(e.g., Sarcoptes scabiei*); and mange mites, which include *Psoroptidae* spp. (*e.g., Chorioptes bovis* and *Psoroptes ovis*).
iii. *Prostigmata* spp, such as chiggers, which include *Trombiculidae* spp. *(e.g.,* North American chiggers, *Trombicula alfreddugesi*).
iv. Demodex.

C. **Ticks.** These include, for example, soft-bodied ticks, such as *Argasidae* spp. *(e.g., Argas* spp. and *Ornithodoros* spp.); and hard-bodied ticks, such as *Ixodidae* spp. (*e.g., Ixodes ricinus, Rhipicephalus sanguineus, Haemaphysalis* spp, *Dermacentor reticulates, Dermacentor variabilis, Amblyomma americanum,* and *Boophilus* spp.).

D. **Lice.** These include, for example, chewing lice, such as *Menopon* spp. and *Bovicola* spp.; and sucking lice, such as *Haematopinus* spp., *Linognathus* spp., and *Solenopotes* spp.

E. **Fleas.** These include, for example, *Ctenocephalides* spp., such as dog fleas (*Ctenocephalides canis*) and cat fleas (*Ctenocephalides felis*); *Xenopsylla* spp., such as oriental rat fleas (*Xenopsylla cheopis*); *Pulex* spp., such as human fleas (*Pulex irritans*); hedgehog fleas *(Archaeopsylla erinacei);* and bird fleas *(Ceratophyllus gallinae*).

F. **True bugs.** These include, for example, *Cimicidae* or the common bed bug *(Cimex lectularius);* and *Triatominae* spp., such as triatomid bugs (also known as kissing bugs) (*e.g., Rhodnius prolixus* and *Triatoma* spp.).

An "infestation" refers to the presence of parasites in numbers that pose a risk of nuisance or harm to humans or animals. The presence can be in the environment (*e.g.*, in animal bedding), on the skin or fur of an animal, etc. Unless otherwise stated, when the infestation is within an animal (*e.g.*, in the blood or other internal tissues), the term infestation is intended to be synonymous with the term, "infection," as that term is generally understood in the art.

The phrase "control of ectoparasite infestation" means to reduce or eradicate parasite numbers in and/or on an animal, and/or to partially or completely inhibit the development of parasite infestation in and/or on an animal. This may be achieved by, for example, killing, repelling, expelling, incapacitating, deterring, eliminating, alleviating, or minimizing the parasite. The control of ectoparasites can be insecticidal and/or acaricidal. The effect of the isoxazoline can be, for example, ovicidal, larvicidal, nymphicidal, adulticidal, or a combination thereof. In addition, the effect can manifest itself directly by killing the parasites either immediately or after some time has elapsed (*e.g.*, when molting occurs or by destroying eggs). The effect alternatively (or additionally) can manifest itself indirectly by, for example, reducing the number of eggs laid and/or the hatch rate.

In general, an amount of a isoxazoline that is sufficient to "control" or be "effective" against a target parasite is an amount that is sufficient to reduce or eradicate parasite numbers in and/or on an animal, and/or to partially or completely inhibit the development of parasite infestation in and/or on an animal. When the isoxazoline is administered systemically, an effective amount generally constitutes an amount that results in tissue and/or blood concentrations generally toxic when ingested by a target parasite.

One of ordinary skill in the art typically can determine an "effective" dose by, for example, observing or detecting changes in a clinical condition or behavior of a host animal, as well as by observing or detecting relative changes in parasite numbers after such treatment. In general, a dose is considered effective for controlling a target parasite when the dose is sufficient to cause an existing or potential target parasite count to be reduced by at least about 5%. In some such instances, for example, the dose is considered effective when the dose is sufficient to cause an existing or potential parasite count to be reduced by at least about 10% (or at least about 30%, at least about 50%, at least about 60%, at least about 75%, at least about 90%, at least about 95%, or at least about 99%).

The optimum dosage generally depends on multiple factors, including, for example, the particular isoxazoline; the identity of any other active ingredient(s) being administered to the animal recipient; the route of administration; the type and severity of the target condition and pathogen; the type (e.g., species and breed), age, size, sex, diet, activity, and condition of the intended animal recipient; and pharmacological considerations, such as the activity, efficacy, pharmacokinetic, and toxicology profiles of the isoxazoline and other active ingredient(s) being administered to the recipient animal. To the extent multiple active ingredients are administered for combined effects on a target parasite or condition, the amount of each ingredient that constitutes an "effective amount" is an amount that, when combined with the other active ingredients, causes the desired effect.

The isoxazoline of **Formula 11-1** is administered as a single dose that is administered to effectively control a target parasite for a longer duration. In accordance with the invention the single dose is effective to control a target

parasite for at least about 2 months, at least about 3 months, at least about 4 months, or at least about 6 months. In accordance with the invention the frequency of treatments is bi-monthly, quarterly, half yearly, or even longer (e.g., yearly). The durations over which the isoxazoline of **Formula 11-1** tend to be effective against various ectoparasites by systemic administration is surprising. This is particularly true, given that such long activities may, in many instances, be obtained using low doses that are non-toxic to the animal recipients without requiring the use of a controlled-release means. Without being limited to any particular theory, it is hypothesized that this long duration of activity stems from the isoxazolines having particularly high toxicity when ingested by the target parasite.

For many animal recipients, the isoxazoline dose and formulation are chosen to maintain a isoxazoline serum level of at least about 1 ng/ml (e.g., 1 to 50 ng/ml). In general, the amount of isoxazo line administered to the animal recipient is from about 0.001 to about 200 mg/kg body weight. In some embodiments, for example, from about 0.01 to about 200 mg/kg body weight is administered. In other embodiments, for example, from about 0.001 to about 100 mg/kg body weight is administered. In some such embodiments, for example, from about 0.01 to about 100 mg/kg body weight is administered. In other such embodiments, from about 1 to about 30 mg/kg body weight is administered. Greater dosages tend to provide for greater duration of activity.

It is contemplated that the duration of activity of a isoxazoline can be extended even further (or made more consistent) by using a controlled-release formulation or dosage form. For example, the isoxazoline can be administered in microspheres, granules, or implants (e.g., a subcutaneous implant) that release the isoxazoline by, example, diffusion and/or erosion. Use of such a dosage form containing from about 1 and about 50 mg/kg body weight (or from about 10 to about 30 mg/kg body weight, such as about 20 mg/kg of body weight) of the isoxazoline may allow for consistent activity lasting over several months or longer (e.g., a year).

In some embodiments of this invention, a isoxazoline of **Formula 11-1** is administered to treat parasitoses of an animal (or make a medicament to treat parasitoses of an animal). The term "parasitoses" includes pathologic conditions and diseases associated with or caused by one or more ectoparasites directly, such as, for example, anemia and flea allergy dermatitis. It also includes pathologic conditions or diseases associated with caused by one or more vector-transmitted pathogens, such as, for example, Lyme disease, ehrlichiosis (particularly canine ehrlichiosis), and Rocky Mountain spotted fever from vector ticks. The phrase "treatment of parasitoses" means to partially or completely inhibit the development of parasitoses of an animal susceptible to parasitoses, reduce or completely eliminate the symptoms of parasitoses of an animal having parasitoses, and/or partially or completely cure parasitoses of an animal having parasitoses. In general, the treatment of parasitoses is achieved by administering a isoxazoline of **Formula 11-1** to control an ectoparasite infestation.

This invention also relates to treatment methods wherein at least an ancillary goal of controlling ectoparasites in and/or on an animal is to control an ectoparasitic infestation in an environment that is occupied (periodically or continuously) by the animal. In some such embodiments, for example, the animal is a companion animal (*e.g.,* a cat or dog). The environment may be, for example, a house or other shelter; a room; a pen, a stall, or other confinement means; bedding; etc.

The terms "administer" and "administration" refer to the delivery of a isoxazoline of **Formula 11-1**, salt of the isoxazoline, solvate of the isoxazoline or salt, or prodrug of the isoxazoline. In some embodiments of this invention, systemic administration is desirable. "Systemic administration" is an administration at a site remote from a site wherein at least a portion of the target parasites reside. With systemic administration, at least a portion of the isoxazoline reaches the target parasite via the animal recipient's bloodstream, other body fluids (lymph fluids), and/or tissues (e.g., skin or fat tissue). Typically, the parasite ingests the isoxazoline along with the animal recipient's blood, other body fluids, and/or tissue. Systemic administration may be achieved in several forms.

The isoxazoline composition is systemically administered topically using a transdermal formulation (*i.e*., a formulation that passes through the skin). Alternatively (or additionally), the composition may be systemically administered topically via the mucosa. Typical formulations for transdermal and mucosal administration include, for example, pour-ons, spot-ons, dips, sprays, mousses, shampoos, powders, gels, hydrogels, lotions, solutions, creams, ointments, dusting powders, dressings, foams, films, skin patches, limb bands, collars, ear tags, wafers, sponges, fibers, bandages, and microemulsions. The pour-on or spot-on methods, for example, comprise applying the isoxazoline composition to a specific location of the skin or coat, such as on the neck or backbone of the animal. This may be achieved by, for example, applying a swab or drop of the pour-on or spot-on formulation to a relatively small area of the recipient animal's skin or coat (*i.e*., generally no greater than about 10% of the animal recipient's skin or coat). In some embodiments, the isoxazoline is dispersed from the application site to wide areas of the fur due to the spreading nature of the components in the formulation and the animal's movements while, in parallel, being absorbed through the skin and distributed via the animal recipient's fluids and/or tissues.

## *IV. Pharmaceutical compositions*

This invention also is directed to pharmaceutical compositions (or medicaments) comprising a isoxazoline of

**Formula 11-1**, salt of the isoxazoline, solvate of the isoxazoline or salt, or prodrug of the isoxazoline. The compositions also may (and generally will) comprise one or more pharmaceutically-acceptable excipients.

Pharmaceutical compositions of the present invention may be manufactured by, for example, processes known in the art. These processes include, for example, a variety of known mixing, dissolving, granulating, emulsifying, encapsulating, entrapping, and lyophilizing processes. Optimal formulation depends on, for example, the route of administration.

Solid dosage forms, for example, may be prepared by, for example, intimately and uniformly mixing the isoxazoline with fillers, binders, lubricants, glidants, disintegrants, flavoring agents (e.g., sweeteners), buffers, preservatives, pharmaceutical-grade dyes or pigments, and controlled release agents.

Contemplated binders include, for example, gelatin, acacia, and carboxymethyl cellulose.

Contemplated lubricants include, for example, magnesium stearate, stearic acid, and talc.

Contemplated disintegrants include, for example, corn starch, alginic acid, sodium carboxymethylcellulose, and sodium croscarmellose.

Contemplated buffers include, for example, sodium citrate, and magnesium and calcium carbonate and bicarbonate.

Contemplated solvents include, for example, water, petroleum, animal oils, vegetable oils, mineral oil, and synthetic oil. Physiological saline solution or glycols *(e.g.,* ethylene glycol, propylene glycol, or polyethylene glycol) also may be included. The solvent preferably has sufficient chemical properties and quantity to keep the isoxazoline solubilized at temperatures in which the composition is stored and used.

Contemplated viscosity-enhancing agents include, for example, polyethylene, methylcellulose, sodium carboxymethylcellulose, hydroxypropylmethylcellulose, hydroxypropylcellulose, sodium alginate, carbomer, povidone, acacia, guar gum, xanthan gum, tragacanth, methylcellulose, carbomer, xanthan gum, guar gum, povidone, sodium carboxymethylcellulose, magnesium aluminum silicate, carboxyvinyl polymers, carrageenan, hydroxyethyl cellulose, laponite, water-soluble salts of cellulose ethers, natural gums, colloidal magnesium aluminum silicateor finely divided silica, homopolymers of acrylic acid crosslinked with an alkyl ether of pentaerythritol or an alkyl ether of sucrose, and carbomers.

Contemplated surfactants include, for example, polyoxyethylene sorbitan fatty acid esters; polyoxyethylene monoalkyl ethers; sucrose monoesters; lanolin esters and ethers; alkyl sulfate salts; and sodium, potassium, and ammonium salts of fatty acids.

Contemplated preservatives include, for example, phenol, alkyl esters of parahydroxybenzoic acid (e.g., methyl *p*-hydroxybenzoate (or "methylparaben") and propyl *p*-hydroxybenzoate (or "propylparaben")), sorbic acid, o-phenylphenol benzoic acid and the salts thereof, chlorobutanol, benzyl alcohol, thimerosal, phenylmercuric acetate and nitrate, nitromersol, benzalkonium chloride, and cetylpyridinium chloride.

Contemplated stabilizers include, for example, chelating agents and antioxidants.

Solid dosage forms also may comprise, for example, one or more excipients to control the release of the isoxazoline. For example, it is contemplated that the isoxazoline may be dispersed in, for example, hydroxypropylmethyl cellulose.

Topical administration may be achieved using, for example, a concentrated solution, suspension (aqueous or non-aqueous), emulsion (water-in-oil or oil-in-water), or microemulsion comprising a isoxazoline dissolved, suspended, or emulgated in a pharmaceutically-acceptable liquid vehicle. In such embodiments, a crystallization inhibitor optionally may generally be present.

When a liquid formulation is used topically on skin, it can be administered by, for example, pouring on, spreading, rubbing, atomizing, spraying, dipping, bathing, or washing. A pour-on or spot-on formulation, for example, can be poured or atomized onto a limited spot on the skin (typically no greater than about 10% of the skin). In some such embodiments, the formulation allows or facilitates the isoxazoline to penetrate the skin and act on other parts body (*e.g.*, the entire body). Such a pour-on or spot-on formulation can be prepared by dissolving, suspending, or emulsifying the isoxazoline in a suitable skin-fitted solvent or solvent mixture. Other excipients may be included as well, such as, for example, a surfactant, colorant, antioxidant, stabilizer, adhesive, etc. Contemplated solvents include, for example, water, alkanol, glycol, polyethylene glycol, polypropylene glycol, glycerin, benzyl alcohol, phenylethanol, phenoxyethanol, ethyl acetate, butyl acetate, benzyl benzoate, dipropylene glycol monomethyl ether, diethylene glycol monobutyl ether, acetone, methyl ethyl ketone, aromatic and/or aliphatic hydrocarbons, vegetable or synthetic oil, DMF, liquid paraffin, silicone, dimethylacetamide, N-methylpyrrolidone, or 2,2-dimethyl-4-oxy-methylene-1,3-dioxolane.

In some embodiments, a topical formulation (particularly a pour-on or spot-on formulation) comprises a carrier that promotes the absorption or penetration of the isoxazoline through the skin into the blood stream, other bodily fluids (lymph), and/or body tissue (fat tissue). Contemplated examples of dermal penetration enhancers include, for example, dimethylsulfoxide, isopropyl myristate, dipropylene glycol pelargonate, silicone oil, aliphatic esters, triglycerides, and fatty alcohols.

Topical formulations also (or alternatively) may comprise, for example, one or more spreading agents. These substances act as carriers that assist in distributing an active ingredient over the animal recipient's coat or skin. They

may include, for example, isopropyl myristate, dipropylene glycol pelargonate, silicone oils, fatty acid esters, triglycerides, and/or fatty alcohols. Various spreading oil/solvent combinations also may be suitable, such as, for example, oily solutions, alcoholic and isopropanolic solutions (*e.g.*, solutions of 2-octyl dodecanol or oleyl alcohol), solutions of esters of monocarboxylic acids (*e.g.*, isopropyl myristate, isopropyl palmitate, lauric acid oxalic ester, oleic acid oleyl ester, oleic acid decyl ester, hexyl laurate, oleyl oleate, decyl oleate, and caproic acid esters of saturated fatty alcohols having a carbon chain of 12 to 18 carbons), solutions of esters of dicarboxylic acids (*e.g.*, dibutyl phthalate, diisopropyl isophthalate, adipic acid diisopropyl ester, and di-n-butyl adipate), or solutions of esters of aliphatic acids (*e.g.,* glycols). When the formulation comprises a spreading agent, it also may be advantageous to include a dispersant, such as, for example, pyrrolidin-2-one, N-alkylpyrrolidin-2-one, acetone, polyethylene glycol or an ether or ester thereof, propylene glycol, or synthetic triglycerides.

When formulated in, for example, an ointment, it is contemplated that the isoxazoline may be mixed with, for example, either a paraffinic or a water-miscible ointment base. When formulated in a cream, it is contemplated that the isoxazoline may be formulated with, for example, an oil-in-water cream base. In some instances, the aqueous phase of the cream base includes, for example at least about 30% (w/w) of a polyhydric alcohol, such as propylene glycol, butane-1,3-diol, mannitol, sorbitol, glycerol, polyethylene glycol, or a mixture thereof.

Other inert ingredients may generally be added to the composition as desired. To illustrate, it is contemplated that these may include, for example, lactose, mannitol, sorbitol, calcium carbonate, sodium carbonate, tribasic calcium phosphate, dibasic calcium phosphate, sodium phosphate, kaolin, compressible sugar, starch, calcium sulfate, dextro or microcrystalline cellulose, colloidal silicon dioxide, starch, sodium starch glycolate, crospovidone, microcrystalline cellulose, tragacanth, hydroxypropylcellulose, pregelatinized starch, povidone, ethylcellulose, hydroxypropylcellulose, hydroxypropylmethylcellulose, and methylcellulose.

A general discussion regarding formulation of drugs and various excipients may be found in, for example, Gennaro, A.R., et al., eds., Remington: The Science and Practice of Pharmacy (Lippincott Williams & Wilkins, 20th Ed., 2000). Another general discussion regarding formulation of drugs and various excipients may be found in, for example, Liberman, H. A., et al., eds., Pharmaceutical Dosage Forms (Marcel Decker, New York, N.Y., 1980).

The concentration of the isoxazoline **Formula 11-1** (or any salt of the isoxazoline, solvate of the isoxazoline or salt, or prodrug of the isoxazoline) in the composition may vary widely depending on, for example, the mode of administration. In general, the concentration is from about 1 to about 70% (by weight). In some such embodiments, for example, the concentration is from about 1 to about 50% (by weight), or from about 10 to about 50% (by weight). In other embodiments, the concentration is from about 35 to about 65% (by weight), from about 40 to about 60% (by weight), from about 45 to about 55% (by weight), or about 50% (by weight).

### *V. Examples of contemplate combination therapies*

The methods disclosed herein encompass methods wherein a isoxazoline is the sole active ingredient administered to the recipient animal. It is contemplated, however, that the methods also encompass combination therapies wherein a isoxazoline is administered in combination with one or more other active ingredients. The other active ingredient(s) may be, for example, one or more other isoxazolines. Alternatively (or additionally), the other active ingredient (s) may be one or more compounds that are not isoxazolines. The other active ingredient(s) may target the same and/or different pathogens and conditions.

Contemplated active ingredient(s) that may be administered in combination with the isoxazoline include, for example, anthelmintics, insecticides and acaricides, insect growth regulators and juvenile hormone analogues, anti-inflammatories, anti-infectives, hormones, dermatological preparations (*e.g.*, antiseptics and disinfectants), and immunobiologicals (*e.g.*, vaccines and antisera) for disease prevention.

Anthelmintics include, for example, avermectins (*e.g.*, ivermectin, moxidectin, and milbemycin), benzimidazoles (*e.g.*, fenbendazole, albendazole, and triclabendazole), salicylanilides (*e.g.*, closantel and oxyclozanide), substituted phenols (*e.g.*, nitroxynil), pyrimidines *(e.g.,* pyrantel), imidazothiazoles (*e.g.*, levamisole), cyclooctadepsipeptide (*e.g.*, Emodepside), and tetrahydropyrimidines (*e.g.,* praziquantel). Anthelmintics also includes, for example, amino acetonitrile derivatives, such as, for example, those discussed in Kaminsky, R., et al., "A new class of anthelmintics effective against drug-resistant nematodes," Nature, vol. 452, pp. 176-180 (March 13, 2008); and Int'l Patent Publ. Nos. WO2006/050887 and WO2005/044784.

The isoxazoline can be according to the invention administered in combination with (and, in some instances, in the same composition with) one or more macrocyclic lactone endectocidal parasiticides. These parasiticides tend to be useful against, for example, a broad spectrum of endoparasites and ectoparasites in mammals.

One particularly contemplated macrocyclic lactone parasiticide is ivermectin. Ivermectin is a semi-synthetic derivative of avermectin, and is generally produced as a mixture of at least 80% 22,23-dihydroavermectin $B1_a$ and less than 20% 22,23-dihydroavermectin $B1_b$. Ivermectin is disclosed in US Patent 4,199,569. Ivermectin has been used as an antiparasitic agent to treat various parasitic diseases since the mid-1980's.

Other macrocyclic lactone parasiticides include, for example:

A. **Abamectin.** This compound is, for example, identified as avermectin $B1_a$/$B1_b$ in U.S. Patent 4,310,519. Abamectin contains at least 80% of avermectin $B1_a$, and not more than 20% of avermectin $B1_b$.
B. **Doramectin.** This compound is known as 25-cyclohexyl-avermectin $B_1$. Its structure and preparation are discussed in, for example, US Patent 5,089,480.
C. **Moxidectin.** This compound is discussed in, for example, US Patent. 4,916,154.
D. **Selamectin.** This compound also is known as 25-cyclohexyl-25-de(1-methylpropyl)-5-deoxy-22, 23-dihydro-5-(hydroxyimino)-avermectin B 1 monosaccharide.
E. **Milbemycin.** This compound also is known as B41. It is isolated from the fermentation broth of a Milbemycin-producing strain of *Streptomyces.* The microorganism, fermentation conditions, and isolation procedures are discussed in, for example, US Patents 3,950,360 and 3,984,564.
F. **Emamectin.** This compound also is known as 4"-deoxy-4"-epimethylaminoavermectin $B_1$. Its preparation is discussed in, for example, US Patent Nos. 5,288,710 and 5,399,717. It is a mixture of two homologues, 4"-deoxy-4"-epi-methylaminoavermectin $B1_a$, and 4"-deoxy-4"-epi-methylaminoavermectin $Bl_b$. Salt of emamectin are commonly used. Nonlimiting examples of such salts are those discussed in US Patent 5,288,710, which include salts derived from benzoic acid, substituted benzoic acid, benzenesulfonic acid, citric acid, phosphoric acid, tartaric acid, and maleic acid. A particularly contemplated salt is emamectin benzoate.
G. **Eprinomectin.** This compound is known as 4"-epi-acetylamino-4"-deoxy avermectin $B_1$. It was developed for use in all cattle classes and age groups. It was the first avermectin to generally show broad-spectrum activity against both endo- and ecto-parasites, while also leaving minimal residues in meat and milk. It generally has an additional advantage of being highly potent when delivered topically.

Insecticides and acaricides include, for example, acephate, acetamiprid, acetoprole, amitraz, amidoflumet, avermectin, azadirachtin, azinphos-methyl, bifenthrin, bifenazate, buprofezin, bistrifluron, buprofezin, carbofuran, cartap, chlorfenapyr, chlorfluazuron, chlorantraniliprole), chlorpyrifos, chlorpyrifos-methyl, chromafenozide, clothianidin, cyflumetofen, cyfluthiin, β-cyfluthrin, cyhalothrin, γ-cyhalothrin λ-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim, flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, monocrotophos, methoxyfenozide, monocrotophos, nitenpyram, nithiazine, novaluron, noviflumuron, óxamyl, parathion, parathiori-methyl, permethrin, phorate, phosalone, phosmet; phosphamidori, pirimicarb, profenofos, profluthrin, protrifenbute, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen, spirotetramat, sulprofos, tebufenozide, teflubenzuron, tefluthrin, terbufos, tetrachlorvinphos, thiacloprid, thiamethoxam, thiodicarb, thiosultap-sodium, tolfenpyrad, tralomethrin, triazamate, trichlorfon, and triflumuron. General references discussing antiparasitic agents, such as insecticides and acaricides, include, for example, The Pesticide Manual, 13th Edition, C. D. S. Tomlin, Ed., British Crop Protection Council, Farnham, Surrey, U.K. (2003).

In some of the methods disclosed herein, the isoxazoline can be administered with pyridylmethylamine derivatives, such as, for example, pyridylmethylamine derivatives discussed in European Patent Appl. EP0539588 or Int'l Patent Appl. Publ. WO2007/115643.

In some of the methods disclosed herein the isoxazo line can be administered with nodulisporic acids and derivatives thereof, such as, for example, compounds discussed in US Patent 5,399,582; 5,945,317; 5,962,499; 5,834,260; 6,221,894; or 5,595,991; or Int'l Patent Appl. Publ. 1996/29073.

Insect growth regulators include, for example, agridyne, diofenolan, fenoxycarb, hydroprene, kinoprene, methoprene, pyriproxyfen, tetrahydroazadirachtin, chlorfluazuron, cyromazine, diflubenzuron, fluazuron, flucycloxuron, flufenoxuron, hexaflumuron, ifenuron, tebufenozide, and triflumuron. These compounds tend to provide both initial and sustained control of parasites at all stages of insect development, including eggs, on the animal subject, as well as within the environment of the animal subject.

Other antiparasitic compounds contemplated to be useful in combination therapies with the isoxazoline include, for example, imidazo[1,2-b] pyridazine compounds discussed in US Patent Appl. Publ. No. 2005-0182059; 1-(4-Mono and di-halomethylsulphonylphenyl)-2-acylamino-3-fluoropropanol compounds discussed US Patent 7,361,689; trifluoromethanesulfonanilide oxime ether compounds discussed in US Patent 7,312,248; n-[(phenyloxy)phenyl]-1,1,1-trifluoromethanesulfonamide and n-[(phenylsulfanyl)phenyl]-1,1,1-trifluoromethanesulfonamide compounds discussed in US Patent Appl. Publ. 2006-0281695; and 2-phenyl-3-(1H-pyrrol-2-yl)acrylonitrile compounds discussed in US Appl. Publ. 2006/0128779.

Anti-inflammatory agents include, for example, corticosteroids, which, in turn, include, for example, beclomethasone dipropionate, betamethasone diproprionate, betamethasone valerate, budesonide, ciclesonide, deflazacort, dexamethasone, fluocinolone acetonide, fluticasone, propionate, fluticasone furoate, loteprednol, etabonate, mometasone, and mometasone furoate, methylprednisolone, prednisolone, prednisone, rofleponide, and triamcinolone acetonide. Anti-inflammatory agents also include, for example, one or more non-steroidal anti-inflammatory drugs ("NSAIDs"). NSAIDs include, for example, salicylates, arylalkanoic acids, 2-arylpropionic acids (or "profens"), N-arylanthranilic acids, pyrazolidine derivatives, oxicams, COX-2 inhibitors, sulphonanilides, and licofelone. Anti-inflammatory ingredients also may include, for example, antihistamines. Antihistamines include, for example, $H_1$-receptor agonists, $H_2$-receptor agonists, $H_3$-receptor agonists, $H_4$-receptor agonists, mast cell stabilizers, and vitamin C.

In the contemplated combination therapies, the isoxazoline of **Formula 11-1** may be administered before, simultaneously, and/or after the other active ingredient(s). In addition, the isoxazoline may be administered in the same composition as the other active ingredient(s) and/or in a separate compositions from the other active ingredient(s). Further, the isoxazoline and other active ingredient(s) may be administered via the same and/or different routes of administration.

When the isoxazoline according to the invention is administered in a combination therapy, the weight ratio of the active ingredients may vary widely. Factors influencing this ratio include, for example, the particular isoxazoline; the identity of the other active ingredient(s) be administered in the combination therapy; the mode(s) of administration of the isoxazoline and other active ingredient(s); the target condition and pathogen; the type (e.g., species and breed), age, size, sex, diet, activity, and condition of the intended recipient; and pharmacological considerations, such as the activity, efficacy, pharmacokinetic, and toxicology profiles of the isoxazoline and other active ingredient(s). In some contemplated embodiments, for example, the weight radio of the isoxazoline to the other active ingredient(s) is, for example, is from about 1:3000 to about 3000:1. In some such instances, the weight ratio is from about 1:300 to about 300:1. In other such instances, the weight ratio is from about 1:30 and about 30:1.

In addition to other active ingredients, it is contemplated that the isoxazoline according to the invention may be administered with one or more other compounds that beneficially affects (e.g., enhances or prolongs) the activity (or other characteristic, such as safety) of the isoxazoline. For example, it is contemplated that the isoxazoline may be administered with one or more synergists, such as, for example, piperonyl butoxide (PBO) and triphenyl phosphate (TPP). Other synergists include, for example, N-(2-ethylhexyl)-8,9,10-trinorborn-5-ene-2,3-dicarboxamide (also known as "ENT 8184" or "MGK 264") and Verbutin (also known as "MB-599"). A discussion relating to insecticidal synergists may be found in, for example, *The Pesticide Manual,* 13th Edition, cited above.

## EXAMPLES

The following examples are merely illustrative, and not limiting to the remainder of this disclosure in any way. The compound numbers referenced in these examples refer to the compound numbers for the structures in the above detailed description and below claims.

**Example 1. Efficacy of Compound 11-1 against cat fleas (*Ctenocephalides felis*).**

Adult fleas (20-30) were fed on artificial membranes with blood spiked with Compound 11-1, or a positive control (fipronil) at a concentration of 100, 10, or 1 ppm. Flea efficacy was assessed after 48 hr of continuous feeding by comparing the number of killed and damaged fleas with the number of fed fleas. Flea efficacy was 100% for Compound 11-1, and fipronil at 100, 10, and 1 ppm.

**Example 2. Model animal studies for Compound 11-1.**

The objective of these studies was to assess the efficacy of Compound 11-1 against various parasites. The parasites were:

| Arthropoda | Species | Family | Common name |
|---|---|---|---|
| Insecta | *Ctenocephalides felis* | Pulicidae | Cat flea |
| Insecta | *Cimex lectularius* | Cimicidae | Bed bug |
| Acari | *Ornithodoros moubata* | Argasidae | Chicken tick |
| Acari | *Rhipicephalus sanguineus* | Ixodidae | Brown dog tick |
| Acari | *Myocoptes musculinus* | Myocoptidae | Rodent fur mite |

### A. ***Efficacy against fleas on mice***

Mice were divided into groups of three, and treated topically with 100 ppm body weight, orally with 10 mg/kg bodyweight, or subcutaneously with 10 mg/kg bodyweight of Compound 11-1, fipronil (positive control), or nothing

(negative control). These mice were sedated and infested with adult fleas *(C. felis)* 1, 3, 6, and 24 hr after treatment. Fleas were recovered from the mice after approximately 30 minutes of feeding. The assessment of flea inhibition (% of dead and damaged fleas) was conducted 1 and 24 hr after each infestation. The results are shown in **Table 1.**

**Table 1**

| **Results for Experiment Assessing *In Vivo* Efficacy of Compound 11-1 Against Fleas on Mice** | | | | |
|---|---|---|---|---|
| | **Time of Infestation Following Treatment** | | | |
| | **1 hr** | **3 hr** | **6 hr** | **24 hr** |
| **Flea inhibition using oral * administration of Compound 11-1 at 1 hr after infestation** | 43.3% | 44.3% | 17.7% | 37.7% |
| **Flea inhibition using subcutaneous * administration of Compound 11-1 at 1 hr after infestation** | 7.7% | 31.0% | 21.0% | 100% |
| **Flea inhibition using topical administration of Compound 11-1 at 1 hr after infestation** | 0% | 0% | 0% | 2% |
| **Inhibition using oral * administration of Compound 11-1 at 24 hr after infestation** | 67.7% | 48.7% | 26.7% | 82.0% |
| **Inhibition using subcutaneous * administration of Compound 11-1 at 24 hr after infestation** | 40.0% | 42.3% | 36.7% | 100% |
| **Inhibition using topical administration of Compound 11-1 at 24 hr after infestation** | 97.7% | 99.0% | 81.0% | 93.3% |
| **Inhibition using oral administration * of fipronil at 1 hr after infestation** | 0-2% | 0-2% | 0-2% | 0-2% |
| **Inhibition using subcutaneous * administration of fipronil at 1 hr after infestation** | 0-2% | 0-2% | 0-2% | 0-2% |
| **inhibition using topical administration of fipronil at 1 hr after infestation** | 0-2% | 0-2% | 0-2% | 0-2% |
| **Inhibition using * oral administration of fipronil at 24 hr after infestation** | 3.3% | 12.3% | 6.7% | 7.7% |
| **Inhibition using subcutaneous * administration of fipronil at 24 hr after infestation** | 1.0% | 15.7% | 97.7% | 94.3% |
| **Inhibition using topical administration of fipronil at 24 hr after infestation** | 100% | 94.3% | 100% | 76.7% |
| **Inhibition of negative control at 1 hr after infestation** | none detected | none detected | none detected | none detected |
| **Inhibition of negative control at 24 hr after infestation** | none detected | none detected | none detected | none detected |
| * comparative examples | | | | |

In general, with Compound 11-1, only a few damaged fleas were found relative to killed fleas. With fipronil, however, killed and damaged fleas were generally found in equal amounts. No side effects were observed with Compound 11-1 or fipronil during this experiment.

### *B. Efficacy against mites on mice*

All mice used in the study had a present mite infestation with *M. musculinus* consisting of all stages of the parasite with at least a medium ("++") infestation rate. The mice were divided into groups of three, and treated topically with 100 ppm body weight, orally with 10 mg/kg body weight, or subcutaneously with 10 mg/kg body weight of Compound 11-1, fipronil (positive control), or nothing (negative control). This treatment was then repeated 7 days later. The infestation rate per mouse ("+" = low infestation rate, "++" = medium infestation rate, "+++" = high infestation rate) was assessed repeatedly on days 1, 6, 9, 13, and 23. Efficacy was defined as the inhibition of the mite infestation rate on treated mice relative to the negative control group. The results are shown in **Table 2.**

**Table 2**

| **Results for Experiment Assessing *In Vivo* Efficacy of Compound 11-1 Against Mites on Mice** | | | | | | |
|---|---|---|---|---|---|---|
| | **Day 0** | **Day 1** | **Day 6** | **Day 9** | **Day 13** | **Day 23** |
| **Infestation after oral administration of Compound 11-1 *** | ++ to +++ | + to ++ (remaining mites slightly damaged) | ++ | 2 mice: none detected<br>1 mouse: + (remaining mites damaged) | none detected | none detected |
| **Infestation after subcutaneous administration of Compound 11-1 *** | ++ to +++ | + to ++ (remaining mites slightly damaged) | ++ | none detected | none detected | none detected |
| **Infestation after topical administration of Compound 11-1** | ++ to +++ | + (remaining mites severely damaged) | 2 mice: none detected<br>1 mouse: + (remaining mites damaged) | none detected | none detected | none detected |
| **Infestation after oral administration of fipronil** | ++ to +++ | +++ | ++ to +++ | ++ to +++ | + | + |
| **Infestation after subcutaneous administration of fipronil *** | ++ to +++ | +++ | 2 mice: + to ++<br>1 mouse: +++ | 2 mice: + to ++<br>1 mouse: +++ | + | + to ++ |
| **Infestation after topical administration of fipronil** | ++ to +++ | 2 mice: + to ++<br>1 mouse: +++ (all mites damaged) | + to ++ | 2 mice: none detected<br>1 mouse: + (remaining mites damaged) | none detected | none detected |
| **Infestation with negative control** | ++ to +++ | ++ to +++ | ++ to +++ | ++ to +++ | ++ to +++ | ++ to +++ |
| * comparative example | | | | | | |

No side effects were observed with Compound 11-1 or fipronil during the mite efficacy study.

***C. Efficacy against hard (ixodide) ticks on Guinea pigs***

Guinea pigs were divided into groups of three. Each group was subjected to one of the following treatments with either Compound 11-1, fipronil (positive control), or neither (negative control):

Study 1:

1. 100 ppm body weight topical administration by animal dip
2. 10 mg/kg body weight oral administration *
3. 10 mg/kg body weight subcutaneous administration *
4. No treatment (negative control)

Study 2:

1. 25 ppm body weight topical administration by animal dip
2. 2.5 mg/kg body weight oral administration *
3. 2.5 mg/kg body weight subcutaneous administration *
4. No treatment (negative control)

* comparative example
One day before the treatment, all the Guinea pigs were infested with 100 vital juvenile ("nymph") hard ticks (brown dog ticks, *R. sanguineus).* Engorged, detached nymphs were counted (eN) from Day 4 to Day 8 to calculate the efficacy of the treatments according to following formula:

$$\text{Efficacy on engorged nymphs (\%)} = \frac{(\sum \text{eN control group} - \sum \text{eN treatment group})}{\sum \text{eN control group}} \text{x100}$$

In addition, collected ticks were assessed for molting into the next stage.

In the first study, 32 engorged *R. sanguineus* nymphs were collected from animals of the untreated negative control group. And in the second study, 75 engorged *R. sanguineus* nymphs were collected from animals of the untreated negative control group. Molting into the next stage was observed for the negative control groups of both studies. In contrast, no nymphs were collected from any of the groups treated with Compound 11-1 or fipronil in either study. Thus, the inhibition of engorged nymphs for both Compound 11-1 and fipronil was 100%. No side effects were observed with Compound 11-1 or fipronil during either study.

**Example 3. Efficacy of Compound 11-1 against cat fleas (*C. felis*) and brown dog ticks (*R. sanguineus*) on dogs.**

On Day Zero, one group of 4 beagles was treated orally with gelatin capsules containing 20 mg/kg body weight of Compound 11-1. Another group of 4 beagles was treated topically by washing with 2 L of solution containing 200 ppm body weight of Compound 11-1 dissolved in DMF-premix/tap water (1:100, based on volume). Finally, a group of 3 beagles remained untreated as the negative control. Two days before treatment on Day Zero, all the beagles were each infested with approximately 80 unfed adult fleas *(C. felis)* and approximately 60 unfed adult ticks *(R. sanguineus).* The parasite burden of each beagle was assessed on Day 2 (approximately 48 hr after the treatment) by removing and counting the fleas and ticks. Fleas and ticks were classified according to vitality (fleas: dead or alive; ticks: dead or alive, and engorged or not engorged). The efficacy was calculated from the mean number of vital fleas and ticks in the treated group (Mt) relative to the mean number of vital fleas and ticks in the untreated control group (Mc) using following formula:

$$\text{Flea / tick efficacy (\%)} = \frac{(Mc - Mt)}{Mc} \text{x100}$$

Compound 11-1 exhibited a flea and tick efficacy of 100% after oral and topical treatment. No side effects were observed during this study.

**Example 4. Further efficacy study of Compound 11-1 against cat fleas (*C. felis*) and brown dog ticks (*R. sanguineus*) on dogs.**

Beagles were randomly assigned to 5 treatment groups of 4 animals each, and one untreated control group of 3 animals. The dogs in the treatment groups were treated on Day Zero as shown in **Table 3**

**Table 3**

| **Treatment Groups for Efficacy Study of Compound 11-1 Against Cat Fleas (*C. felis)* and Brown Dog Ticks (*R. sanguineus*) on Beagles** | |
|---|---|
| **Group** | **Treatment** |
| **A** | *1 mg/kg body weight Compound 11-1 in the form of a tablet for oral * administration.* The composition was 13.33% Compound 11-1; 3.29% lactose monohydrate (Granulac 200); 0.01% of sodium lauryl sulfate; 0.90% Povidone 25; 0.15% water; 40.98% lactose monohydrate (Flowlac 100); 33.33% corn starch; 3.00% silica, colloidal anhydrous; 4.00% sodium starch glycolate; and 1.00% magnesium stearate. |
| **B** | *1 mg/kg body weight Compound 11-1 in the form of an injectable solution for * subcutaneous administration.* One ml of the composition contained 20 mg of Compound 11-1; 0.4 ml of dimethyl sulfoxide;0.24 ml of propylene glycol; and sufficient ethyl lactate to bring the total volume to 1 ml. |
| **C** | *1 mg/kg body weight Compound 11-1 in the form of a topical solution for spot-on administration (the solution included an absorption enhancer).* One ml of the composition contained 20 mg of Compound 11-1; 0.35 ml of dimethyl sulfoxide; and sufficient dipropylene glycol monomethyl ether to bring the total volume to 1 ml. |
| **D** | *1 mg/kg body weight Compound 11-1 in the form of a topical solution for spot-on administration (the solution included an absorption enhancer and spreading agent).* One ml of the composition contained 20 mg of Compound 11-1; 0.35 ml of dimethyl sulfoxide; 0.1 ml of isopropyl myritate; and sufficient dipropylene glycol monomethyl ether to bring the total volume to 1 ml. |
| **E** | *1 mg/kg body weight Compound 11-1 in the form of a topical solution for spot-on administration.* One ml of the composition contained 20 mg of Compound 11-1; and sufficient ethyl lactate to bring the total volume to 1 ml. |
| **F** | *None (negative control)* |
| * comparative example | |

The dogs were infested with approximately 80 fleas (*C. felis*) and 60 ticks (*R. sanguineus*) on Days -2, 7, 14, 21, 28, 35, 42, and 49. Fleas and ticks were counted on Day 2 (approximately 48 h after treatment), as well as Days 9, 16, 23, 30, 37, 44, and 51 (approximately 48 hr after each post-treatment re-infestation) to evaluate the insecticidal and acaricidal activity in the treated groups. In Group B, an additional flea and tick infestation was conducted on Day 56, with a respective flea and tick count on Day 58. In addition to the flea and tick counts, blood samples were collected before the treatments, as well as approximately 2 hr, 4 hr, 8 hr, 24 hr, and 72 hr after the treatment on Day Zero, and then once on each of Days 7, 14, 21, 28, 35, 42, 49, and 56. The concentration of Compound 11-1 in blood plasma was analyzed by HPLC/MS/MS. The LOQ of this method was 5 ng/ml. **Table 8** shows the observed tick efficacies:

**Table 4**

| **Efficacies of Various Treatments with Compound 11-1 Against Brown Dog Ticks (*R. sanguineus*) on Beagles** | | | | | | | | | |
|---|---|---|---|---|---|---|---|---|---|
| **Group** | **Day** 2 | **Day 9** | **Day 16** | **Day 23** | **Day 30** | **Day 37** | **Day 44** | **Day 51** | **Day 58** |
| **A** | 99.4 | 99.3 | 98.3 | 98.2 | 98.9 | 94.6 | 82.0 | 82.0 | |
| **B** | 100 | 99.3 | 100 | 98.8 | 99.5 | 98.8 | 95.1 | 88.8 | 74.0 |
| **C** | 81.9 | 98.7 | 94.8 | 98.2 | 96.2 | 87.0 | 76.5 | 55.3 | |
| **D** | 83.1 | 96.1 | 98.3 | 98.2 | 96.2 | 94.1 | 73.8 | 63.4 | |
| **E** | 70.6 | 90.2 | 95.9 | 91.5 | 97.8 | 89.2 | 79.8 | 59.6 | |

(continued)

| Efficacies of Various Treatments with Compound 11-1 Against Brown Dog Ticks (*R. sanguineus*) on Beagles | | | | | | | | | |
|---|---|---|---|---|---|---|---|---|---|
| **Group** | **Day** 2 | **Day 9** | **Day 16** | **Day 23** | **Day 30** | **Day 37** | **Day 44** | **Day 51** | **Day 58** |
| **F** | 0 | 0 | 0 | 0 | 0 | 0 | 0 | 0 | |

**Table 9** shows the observed flea efficacies:

**Table 5**

| Efficacies of Various Treatments with Compound 11-1 Against Cat Fleas (*C. felis*) on Beagles | | | | | | | | | |
|---|---|---|---|---|---|---|---|---|---|
| **Group** | **Day 2** | **Day 9** | **Day 16** | **Day 23** | **Day 30** | **Day 37** | **Day 44** | **Day 51** | **Day 58** |
| **A** | 100 | 100 | 100 | 100 | 100 | 99.6 | 100 | 100 | |
| **B** | 100 | 100 | 100 | 100 | 99.6 | 100 | 100 | 100 | 99.7 |
| **C** | 100 | 100 | 100 | 100 | 100 | 100 | 100 | 100 | |
| **D** | 100 | 100 | 100 | 100 | 100 | 100 | 100 | 100 | |
| **E** | 100 | 100 | 100 | 100 | 100 | 100 | 100 | 100 | |
| **F** | 0 | | 0 | 0 | 0 | 0 | 0 | 0 | |

The observed mean plasma concentration of Compound 11-1 over the duration of the study is shown in **Figure 1.** In **Figure 1,** the **"PO"** data refers to **Group A,** the "SC" data refers to **Group B,** the "TOP W/ ENH" data refers to Group C, the "TOP W/ ENH & SPREAD" data refers to **Group D,** and the "TOP W/ ETHYL LACTATE" data refers to **Group E.** The data for **Group F** (the control) was not included in **Figure 1,** given that there was no Compound 11-1 administered to that group.

**Example 5. Isolation of the R and S enantiomers of Compound 11-1.**

Compound 11-1 (260 mg) was dissolved in a 1:1 mixture of n-hexane/ethanol (13 ml) at 40°C. 80% of this solution was separated into aliquots of 400 μl on a semi-preparative liquid chromatographic system equipped with a Diacel Chiralpak® AD-H column with 250 mm column length, 10 mm diameter, and 5 μm particle size. The mobile phase consisted of a 8:2 mixture of n-hexane/ethanoL A flow rate of 4 ml/min was used. The chiral fractions of both enantiomers were collected and evaporated in vacuum. The purity of the pooled fractions was controlled by analytical chiral chromatography using a Diacel Chiralpak® AD-H column (250 x 4.6 mm, 5 μm) and UV detection at 254 nm. For both enantiomers, a purity of greater than 99% was determined. This technique afforded 88 mg of Compound 17-1 (the S-enantiomer), which had an optical rotation of $[\alpha]_D^{23}$ +63.97° (ethanol, c = 2.97 mg/ml); and 80 mg of Compound 11-1R (the R-enantiomer), which had an optical rotation of $[\alpha]_D^{23}$ -61.07° (ethanol, c = 3.93 mg/ml).

The term "pharmaceutically acceptable" is used adjectivally to mean that the modified noun is appropriate for use in a pharmaceutical product. When it is used, for example, to describe a salt, excipient, or solvate, it characterizes the salt, excipient, or solvate as being compatible with the other ingredients of the composition, and not deleterious to the intended recipient animal to the extent that the deleterious effect(s) outweighs the benefit(s) of the salt, excipient, or solvate.

## Claims

**1.** An isoxazoline of Formula (11-1)

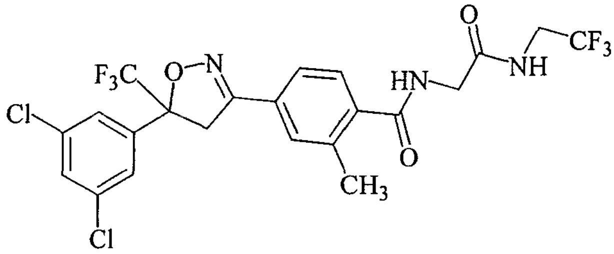

**(11-1)**

for use in the treatment of ectoparasite infestations in animals, wherein a dose of the isoxazoline or a salt of the isoxazoline, or a solvate of the isoxazoline or salt is administered topically at a bi-monthly, quarterly, half-yearly, or longer frequency to the animal.

2. The isoxazoline according to claim 1, wherein a dose is administered topically quarterly to the animal

3. The isoxazoline according to claim 1 or 2, wherein the ectoparasitic infestation comprises a flea infestation.

4. The isoxazoline according to claim 1, 2 or 3, wherein the ectoparasitic infestation comprises a tick infestation.

5. The isoxazoline according to claim 1 to 4, wherein the animal is a cat or dog.

6. An isoxazoline of Formula (11-1)

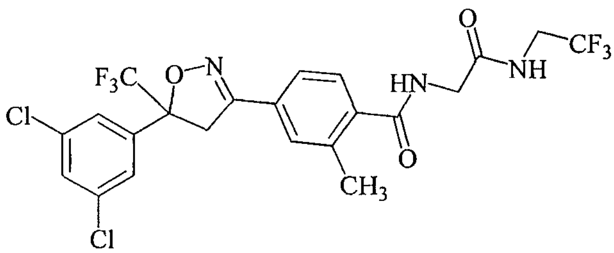

**(11-1)**

or a salt of the isoxazoline, or a solvate of the isoxazoline or salt for use in the treatment of parasitoses of an animal, wherein a dose of the isoxazoline or a salt of the isoxazoline or a solvate of the isoxazoline or salt is administered topically at a bimonthly, quarterly, half-yearly or longer frequency to the animal.

7. The isoxazoline according to claim 6, wherein the parasitoses comprises anemia, flea allergy dermatitis, Lyme disease, ehrlichiosis, or Rocky Mountain spotted fever.

8. A parasiticidal composition for use with an animal comprising a isoxazoline or a salt of the isoxazoline, or a solvate of the isoxazoline or salt in an amount that is effective to control an target parasite when administered topically to the animal , and an excipient; the isoxazoline corresponds in structure to Formula (11-1)

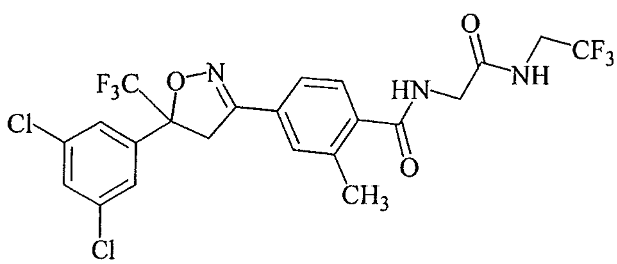

**(11-1),**

wherein the composition is administered at a bimonthly, quarterly, half-yearly or longer frequency.

**Patentansprüche**

1. Isoxazolin der Formel (11-1)

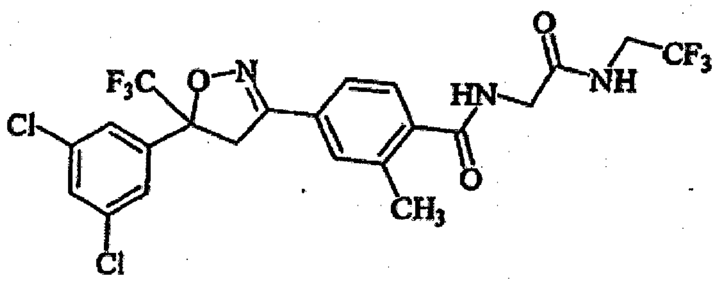

(11-1)

für die Verwendung in der Behandlung von Ektoparasitenbefall bei Tieren, wobei dem Tier eine Dosis des Isoxazolins oder eines Salzes des Isoxazolins oder eines Solvats des Isoxazolins oder Salzes topisch im Abstand von 2 Monaten, einmal pro Vierteljahr, einmal pro halbem Jahr oder in einem längeren Abstand verabreicht wird.

2. Isoxazolin nach Anspruch 1, wobei dem Tier eine Dosis einmal pro Vierteljahr topisch verabreicht wird.

3. Isoxazolin nach Anspruch 1 oder 2, wobei der Ektoparasitenbefall einen Befall mit Flöhen umfasst.

4. Isoxazolin nach Anspruch 1, 2 oder 3, wobei der Ektoparasitenbefall einen Befall mit Zecken umfasst.

5. Isoxazolin nach Anspruch 1 bis 4, wobei es sich bei dem Tier um eine Katze oder einen Hund handelt.

6. Isoxazolin der Formel (11-1)

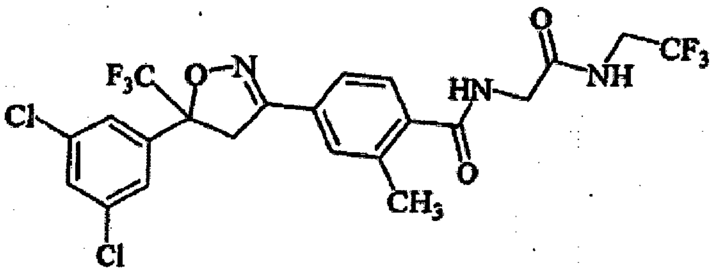

(11-1)

oder ein Salz des Isoxazolins oder ein Solvat des Isoxazolins oder Salzes für die Verwendung in der Behandlung von parasitären Erkrankungen eines Tiers, wobei dem Tier eine Dosis des Isoxazolins oder eines Salzes des Isoxazolins oder eines Solvats des Isoxazolins oder Salzes topisch im Abstand von 2 Monaten, einmal pro Vierteljahr, einmal pro halbem Jahr oder in einem längeren Abstand verabreicht wird.

7. Isoxazolin nach Anspruch 6, wobei die parasitäre Erkrankung Anämie, Flohallergiedermatitis, Lyme-Krankheit, Ehrlichiose oder Rocky-Mountain-Fleckfieber umfasst.

8. Parazitizide Zusammensetzung für die Verwendung bei einem Tier, umfassend ein Isoxazolin oder ein Salz des Isoxazolins oder ein Solvat des Isoxazolins oder Salzes in einer Menge, die wirksam ist, um bei der topischen Verabreichung an das Tier einen Zielparasiten zu bekämpfen, sowie einen Grundstoff; das Isoxazolin entspricht strukturmäßig Formel (11-1)

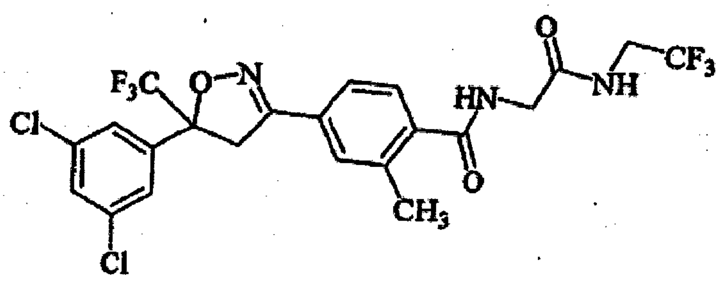

(11-1)

wobei die Zusammensetzung alle zwei Monate, einmal pro Vierteljahr, einmal pro Halbjahr oder in einem längeren Abstand verabreicht wird.

## Revendications

**1.** Isoxazoline de formule (11-1)

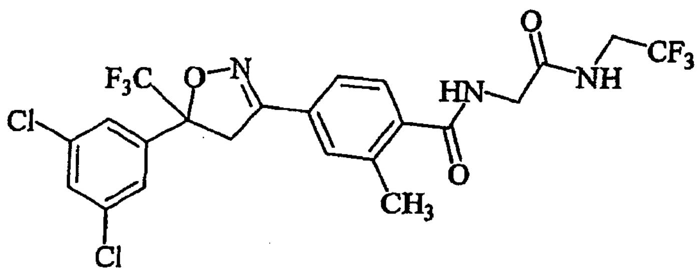

(11-1)

destinée à être utilisée dans le traitement d'infestations ectoparasitaire chez les animaux, une dose de l'isoxazoline ou d'un sel de l'isoxazoline, ou d'un solvate de l'isoxazoline ou du sel étant administrée par voie topique à une fréquence bimensuelle, trimestrielle, semestrielle ou plus longue à l'animal.

**2.** Isoxazoline selon la revendication 1, **caractérisée en ce que** la dose est administrée par voie topique à une fréquence trimestrielle à l'animal.

**3.** Isoxazoline selon la revendication 1 ou 2, **caractérisée en ce que** l'infestation ectoparasitaire comprend une infestation par des puces.

**4.** Isoxazoline selon la revendication 1, 2 ou 3, **caractérisée en ce que** l'infestation ectoparasitaire comprend une infestation par des tiques.

**5.** Isoxazoline selon les revendications 1 à 4, **caractérisée en ce que** l'animal est un chat ou un chien.

**6.** Isoxazoline de formule (11-1)

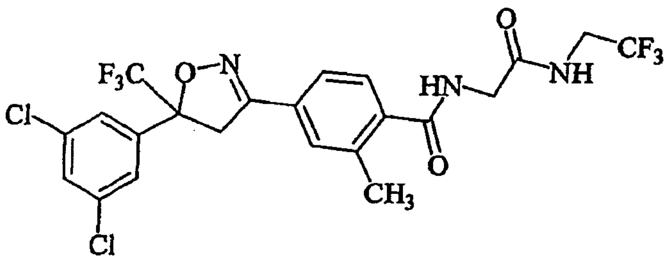

(11-1)

ou un sel de l'isoxazoline ou un solvate de l'isoxazoline ou du sel, destiné à être utilisé dans le traitement de parasitoses chez un animal, la dose de l'isoxazoline ou d'un sel de l'isoxazoline, ou d'un solvate de l'isoxazoline ou du sel étant administrée par voie topique à une fréquence bimensuelle, trimestrielle, semestrielle ou plus longue à l'animal.

**7.** Isoxazoline selon la revendication 6, **caractérisée en ce que** la parasitose comprend l'anémie, l'allergie aux puces, la dermatite, la maladie de Lyme, l'ehrlichiose ou la fièvre pourprée des montagnes rocheuses.

**8.** Composition parasiticide destinée à être utilisée avec un animal, comprenant une isoxazoline ou un sel de l'isoxazoline, ou un solvate de l'isoxazoline ou du sel dans une quantité qui est efficace pour lutter contre un parasite cible lorsqu'elle est administrée par voie topique à l'animal, et un excipient ; la structure de l'isoxazoline correspond à la formule (11-1)

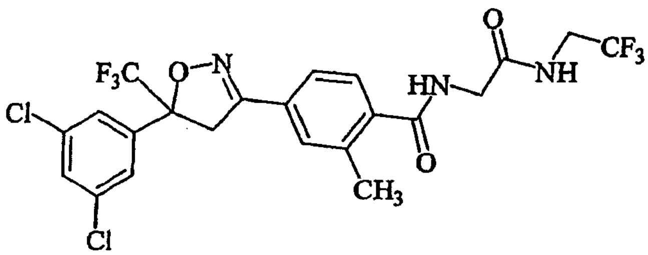

(11-1)

la composition étant administrée à une fréquence bimensuelle, trimestrielle, semestrielle ou plus longue.

# FIGURE 1

**Mean plasma Concentration of Compound 11 -1 During the Study in Example 4**

**Assessing the Efficacy of Compound 11 -1 Against Cat Fleas (*C. felis*) and Brown Dog Ticks (*R. sanguineus*) in Dogs**

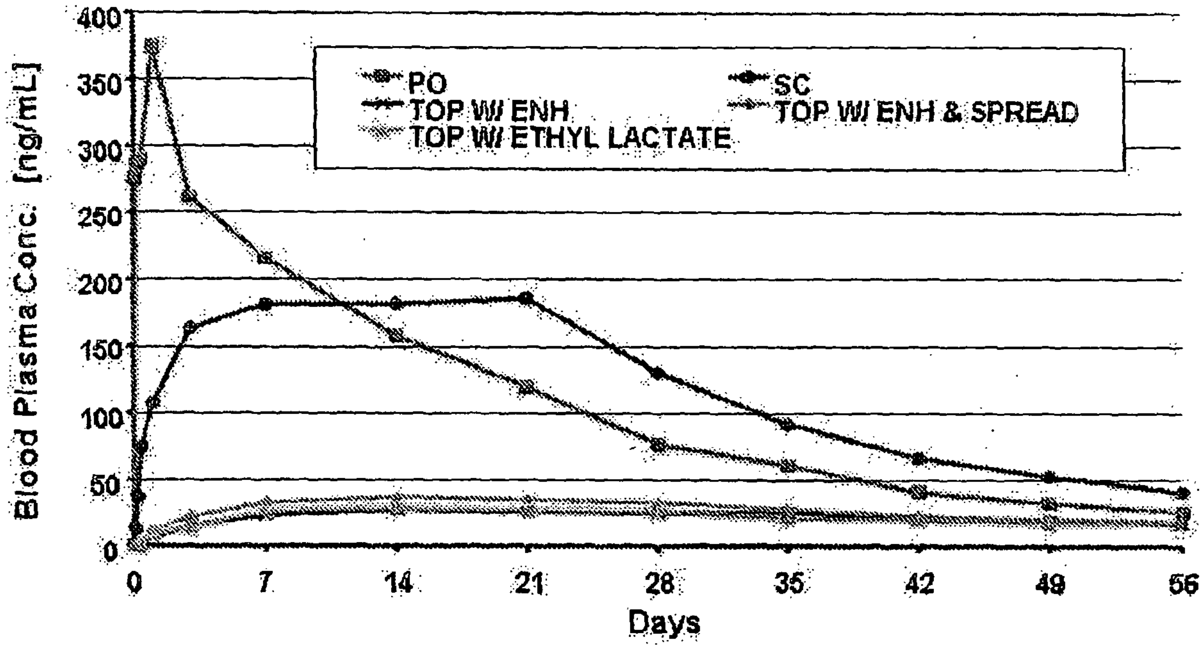

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009003075 A
- US 20070066617 A
- WO 2007026965 A
- JP 2007308471 A
- WO 2005085216 A
- JP 2008054096 W
- EP 1932836 A
- JP 2008133242 A
- WO 2006050887 A
- WO 2005044784 A
- US 4199569 A
- US 4310519 A
- US 5089480 A
- US 4916154 A
- US 3950360 A
- US 3984564 A
- US 5288710 A
- US 5399717 A
- EP 0539588 A
- WO 2007115643 A
- US 5399582 A
- US 5945317 A
- US 5962499 A
- US 5834260 A
- US 6221894 A
- US 5595991 A
- WO 199629073 A
- US 20050182059 A
- US 7361689 B
- US 7312248 B
- US 20060281695 A
- US 20060128779 A


### Non-patent literature cited in the description


- Remington: The Science and Practice of Pharmacy. Lippincott Williams & Wilkins, 2000
- Pharmaceutical Dosage Forms. Marcel Decker, 1980
- **KAMINSKY, R. et al.** A new class of anthelmintics effective against drug-resistant nematodes. *Nature,* 13 March 2008, vol. 452, 176-180
- The Pesticide Manual. British Crop Protection Council, 2003